(19) Europäisches Patentamt
European Patent Office
Office européen des brevets

(11) EP 2 264 630 A1

(12) **EUROPEAN PATENT APPLICATION**

(43) Date of publication:
**22.12.2010 Bulletin 2010/51**

(51) Int Cl.:
***G06F 19/00*** *(2006.01)*

(21) Application number: **10160460.1**

(22) Date of filing: **20.04.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(30) Priority: **20.04.2009 EP 09158291**

(71) Applicant: **Universität Potsdam**
**14469 Potsdam (DE)**

(72) Inventors:
- **Holschneider, Matthias**
  **10713, Berlin (DE)**
- **Schwenk, Bruno**
  **10119, Berlin (DE)**

(74) Representative: **Müller & Schubert**
**Neue Promenade 5**
**10178 Berlin (DE)**

# (54) Method for investigating ternary relationships

(57) The present invention relates to a method for investigating ternary relationships in a set of electronically storable data, wherein planes are investigated, by
a) determining flat prior from a rotational and translational invariance, and then
b) determining the Bayesian posterior in the space of all possible 2 planes in 3 space, and
c) visualisation of the posterior function.

The method of the present invention enriches the toolbox of systems biology with the novel method, called Ex-Planes, for the exploration of ternary relationships in profile data that combines following features: Invariance against rotations and consequently relabeling of the axes, which makes it especially suitable in cases where no a-priori ordering of the variables can be assumed.

*Fig. 3*

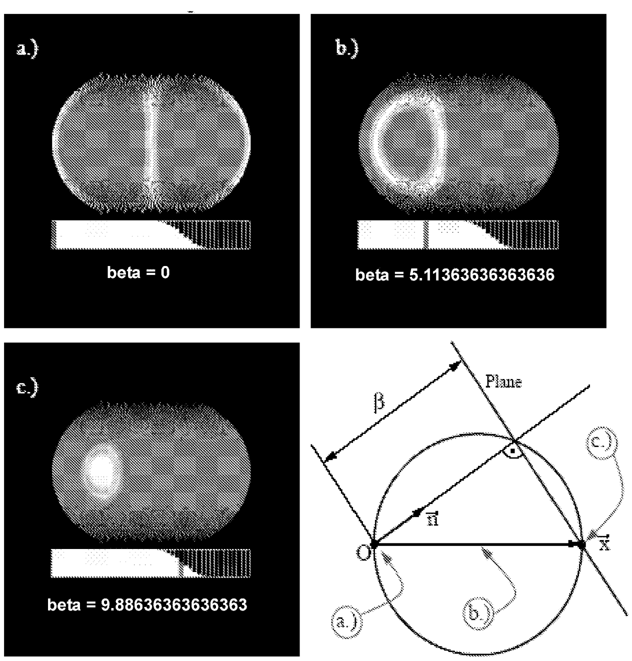


EP 2 264 630 A1

Printed by Jouve, 75001 PARIS (FR)

## Description

The present invention relates to a method for investigating ternary relationships in a set of electronically storable data, wherein planes are investigated.

## Background of the invention

Over the last twenty years, significant advances in experimental technologies have provided the prerequisites for a better understanding of cellular regulatory and metabolic processes, cf. Grünenfelder B, Winzeler E: Treasures and Traps in Genome-Wide Datasets: Case Examples from Yeast. Nat. Rev. Genetics 2002, 3:653-661; Fiehn O, Kopka J, Dörmann P, Altmann T, Trethewey R, Willmitzer L: Metabolite Profiling for Plant Functional Genomics. Nat. Biotechnol. 2000, 18:1157-1161; Pandey A, Mann M: Proteomics to Study Genes and Genomes. Nature 2000, 405:837-846.

These data on gene expression, metabolite concentrations, protein abundances, and also fluxes allow the improvement of pathway and network modelling and simulation with the goal of better understanding the dynamics of cellular processes, cf. Heinrich R, Schuster S: The Regulation of Cellular Systems. New York: Chapman and Hall 1996; Jamshidi N, Palsson B: Formulating Genome-Scale Kinetic Models in the Post-Genome Era. Molecular Systems Biology 2008, 4:171; Stelling J, Klamt S, Bettenbrock K, Schuster S, Gilles E: Metabolic Network Structure Determines Key Aspects of Functionality and Regulation. Nature 2002, 420:190-193.

A variety of methods to analyze these data is based on the detection of local pair-wise (or binary) relationships that also can be used to generate networks which represent defined aspects of structure within the data such that methods from graph theory can be applied to make further inferences on the underlying biology. The traditional way of defining local relations that give rise to global graph-like structure consists in considering pair-wise relationships described by linear or nonlinear distance measures such as Pearson correlation or mutual information, cf. Butte A, Kohane IS: Mutual Information Relevance Networks: Functional Genomic Clustering Using Pair-Wise Entropy Measurements. Pac. Symp. Biocomput. 2000, 5:415-426; Kose F, Budczies J, Holschneider M, Fiehn O: Robust detection and verification of linear relationships to generate metabolic networks using estimates of technical errors. BMC Bioinformatics 2007, 8: 162; Steuer R, Kurths J, Daub C, Selbig J: The Mutual Information: Detecting and Evaluating Dependencies Between Variables. Bioinformatics 2002, 18, Suppl 2:231-240. These approaches allow the identification of highly interconnected sets of molecules that ostensibly form functional units from dependencies that are defined only between pairs of variables.

While these methods only investigate pair-wise relations it is a fundamental fact that interactions between the building blocks of bio systems give rise to relationships of higher than bivariate complexity. One example is the regulation of enzyme activity that induces dependencies not only between product and substrate of an enzyme, but also with possible regulatory active compounds, another is the oligomerization of proteins influencing their biologic activity.

Recently the information processing inequality, partial correlation and conditional mutual information are taken into account in order to represent ternary relationships between triplets of variables and especially the concept of direct causation, cf. Margolin A, I Neemenmann KB, Wiggins C, Stolovitzky G, Favera RD, Califano A: Arcane: An Algorithm for the Reconstruction of Gene Regulatory Networks in a Mammalina Cellular Context. BMC Bioinformatics 2006, 7, Suppl. 1:S7; Yang Y, Tashman A, Lee J, Yoon S, Mao W, Ahn K, Kim W, Mendell N, Gordon D, Finch S: Mixture Modelling of Microarray Gene Expression Data. BMC Proc 2007, 1 Suppl. 1:S 50; Zhao W, Serpedin E, Gougherty E: Inferring Connectivity of Genetic Regulatory Networks Using Information-Theoretic Criteria. IEEE/ACM Transactions on Computational Biology and Bioinformatics 2008, 5:262-274.

## Summary of the invention

According to the present invention a method published by Kose et al. is generalized for investigating binary relationships and propose an approach, that is herein named ExPlanes (Exploring Planes in triplet data), for investigating ternary relationships relying on the Bayesian formalism. It differs from the previous approaches in the state of the art in specific aspects and gives a method to investigate otherwise inaccessible types of interrelations.

While the method according to Kose et al. deals with the investigation of (multiple) 1-dimensional linear relationships in 2-space (geometrically: lines) the method according to the present invention investigates 2- dimensional linear relationships 3-space (geometrically: planes) as the only linear generalization being non trivial in the sense that it cannot be inferred from the knowledge of 2-dimensional projections of the data alone. Therefore, it is an object of the present invention to overcome the backlogs of the state of the art.

The problem is solved by a method according to the main claim of the present invention.

The problem is solved by a method for investigating ternary relationships in a set of electronically storable data, wherein planes are investigated, by

a) determining flat prior from a rotational and translational invariance, and then

b) determining the Bayesian posterior in the space of all possible 2 planes in 3 space, and
c) visualisation of the posterior function.

The method according to the invention is further characterized, in that the Bayesian posterior is based on an error model, which is a mixture of a Gaussian and an uniform distribution.
The method according to the invention is further characterized in that for the visualisation the Bayesian posterior for all directions of the normal vector of the planes is displayed for a fixed beta value and that additionally the beta marginal of the Bayesian posterior is displayed.
The method according to the invention is further characterized in that the given β-value is mapped in the presentation of the β-marginal.
The method according to the invention is further characterized in that the β-value is varied in the presentation of the β-marginal, whereby the presentation of the Bayesian posterior is visualized for the varied β-value.
The method according to the invention is further characterized in that a further step is performed after step b) and before step c), wherein a set of statistical tests is defined, the said tests being based on a family of test statistics that are defined as functional of the posterior, whereby specific hypotheses about the structure being present in the measured data are made detectable.
The method according to the invention is further characterized in that the test functions are selected from curvature based test statistics and concentration based test statistics like entropy or polynomial measures of concentration
The method according to the invention is further characterized in that the result of at least one of the tests is outputted.
The core of the method according to the present invention is the numeric approximation and interactive visualization of the Bayesian posterior of the set of 2-planes with respect to data in 3-space and a robust error model.
In contrast to conventional regression methods it is explicitly required that the results of the method according to the present invention does not depend on the ordering of the variables a- priori. This refers to a situation in which a causal direction between the quantities under investigation is not known a-priori or even impossible to define on an conceptional level. The a-priori independence on ordering is incorporated by creating a translation- ally and rotationally invariant Bayesian posterior in the space of planes, which is subsequently processed and visualized by techniques building on these properties.
From the method in Kose et al. the characteristics inherited are

- Robustness against outliers in the data.
- Use of estimates for measurement errors.
- Capability of exploring multiple linear relationships.
- Testing for specific structures in the data.
  while the generalizations consist in following points:
- Treatment of ternary relationships.
- Interactive visualization of the posterior.
- Invariance against rotation and translation.
- Application to classification problems.

The method according to the invention, called ExPlanes, has three parts: First to compute and interactively visualize the Bayesian posterior in the space of all possible 2-planes in 3- space (two dimensional linear relations) in a translationally and rotationally invariant way. Since the space on which the plane posterior is defined is itself a 3-dimensional manifold it cannot be overseen visually by plotting a single diagram, as it was possible for the case of binary relationships cf. Kose et al. The method of the present invention of visualization uses the mathematical structure of this space, which is the Cartesian product of the surface of the unit sphere and the positive, real numbers to display and explore the posterior interactively. Second to use the posterior to detect specific ternary relationships by defining a family of test statistics as functionals of this posterior distribution. Third to use the plane posterior to perform a classification task employing an stochastical algorithm for optimization, whose results can in turn be used in the process of visualizing and exploring the data.
In contrast to the methods described by Butte et al., Margolin et al., and Zhao et al, that estimate mutual information as difference of entropies indirectly calculated from histogram- or kernel-estimates of the probability density function generating the data, the method of the present invention, ExPlanes, avoids the estimation of entropies. Moreover it incorporates an error model that allows the accommodation of its sensitivity to outliers. While the methods based on (conditional) mutual information do not allow the specification of the type of relation, the method of the present invention allows the testing for specific ternary relationships. Because the method of the present invention is rotationally invariant the results do not depend on the ordering of the variable triplets and is especially suited in situations, in which no causal ordering can be assumed, in contrast to regression methods.

The method of the present invention was applied exemplarily to metabolite profile data generated by microchip-based nanoflow-direct-infusion QTOF mass spectrometry cf. Scholz M, Gatzek S, Sterling A, Fiehn O, Selbig J: Metabolite fingerprinting: detecting biological features by independent component analysis. Bioinformatics 2004, 20(15):2447-2454. In addition to the first results derived by a combination of principal and independent component analysis, the inventors show here that far more detailed information on the level of triplets of single metabolites, in contrast to the processing of aggregated quantities like PCA and ICA, can be obtained. Further the inventors demonstrate that already on the level of metabolite triplets the data are distinctive for the original classification problem.

It is noted, that while the focus of the present descryption is on biological applications, the discussed methodology can be applied for data associated with various other systems. Because of the 3-dimensional data space it seems especially suitable for certain problems in geophysics as the analysis of seismic data from earthquake catalogs in a given geographical region in an effort to clarify the geometry of the underlying structure of the faults which generate the earthquakes (e.g. Bayesian estimation of faults geometry based on seismic catalogue data, Volume 87 of EOS Trans. Amer. Geophys. Union, Fall Meet. Suppl. 2006). The results of such analysis can have fundamental implications for earthquake physics and assessment of seismic hazard associated with a given fault system.

## Detailed Description of the Invention

In the following the inventors will describe the theoretical basis for detecting planes in 3-dimensional data spaces. The inventors will use the framework of Bayesian statistics. Therefore, in a first step the inventors will introduce a suitable parameterization of the space of all 2-planes in 3-space and show that there is a natural nonimformative prior distribution of planes, that is invariant under translations and rotations, which is a natural requirement for a distribution representing the absence of knowledge. The inventors then describe a robust formulation of the posterior distribution given the observations and their individual errors, and interactively visualize it. The inventors show that the way of introducing robustness our method inherits from Kose et al. also makes it possible to explore multiple linear relationships in the data. The inventors investigate the suitability of various functionals of the posterior as test-statistics for detecting plane-related structures. Finally the inventors give an algorithm for supervised learning of a classification problem based on the plane posterior.

## Short description of the Figures

Figure 1 - Plane Definition

Definition of a plane *E* as the set of vectors $\vec{x}$ that have the same projected distance β in direction of a given unit vector $\vec{n}$.

Figure 2 - Thales' Theorem

The set of planes that "explain" a single data point $\vec{x}$, that means which have zero distance to it. The vector $\beta \cdot \vec{n}$, called the *descriptor* of a plane E, forms a rectangular triangle with $\vec{x}$ as hypotenuse. This means the descriptors of all planes explaining a data point form a sphere through $\vec{x}$ and the origin of the coordinate system (Thales' theorem), of which here a cut in two dimensions is shown.

Figure 3 - Visualization

Visualization of the posterior resulting from a single data point $\vec{x}$ = (0, 10, 0). In three screen shots the sphere part of the posterior for values of β corresponding to the origin of the coordinate system (Part a.), the position of the data point itself (Part c.) and an intermediate value are (Part b.) shown. The red arrows in the overview diagram indicate which screen shot belongs to which position in the Thales sphere. The error model was isotropic with σ=1.

Figure 4 - Two Planes

Synthetic data for a mixture of two planes with $\phi = \frac{\pi}{4}$, $\theta = \frac{\pi}{2}$, β=2 (green, 100 data points) and φ=π, $\theta = \frac{\pi}{2}$, β=5 (red, 50 data points). The data points are normally scattered on the planes with a standard deviation of σ=5, in addition isotropic Gaussian noise with standard deviation of σ=1 is added

Figure 5 - Two Planes Posterior

Posterior for the synthetic data in Figure 4. An isotropic error model with standard deviation of σ=1 is assumed, the out lier factor is *c*=1. Part a.) shows the sphere part for β=1.86 and Part b.) for β=4.95, which are approximately the values of the corresponding planes, indicated by blue integer numbers in the map. The bimodality is clearly visible from the sphere part and the β-marginal.

Figure 6 - Entropy

Distribution of the entropy (26) under the null hypothesis $H_0$ "data stray around plane" and alternative hypothesis $H_1$ "data are isotropically scattered in space", see definition (2.7.1), for *N* =17 data points. Histogram sampled over 1200 repetitions.

Figure 7 - Polynomial Measure of Concentration Distribution of the polynomial measure of concentration (27) under the null hypothesis $H_0$ "data stray around plane" and alternative hypothesis $H_0$ "data are isotropically scattered in space", see definition (2.7.1), for *N =17* data points and exponent *q*=0.5. Histogram sampled over 1200 repetitions.

Figure 8 - Curvature based Test-Statistics

Left: Values of mean curvature *M* and Gaussian curvature *G* for data points sampled from different statistical hypotheses, which are: Data stray around a plane (green), a line (blue) and a point (red). Precise mathematical definitions of these hypotheses and parameters are given in section 2.9.2. The plane generated values clearly lie near the $G = M^2$ curve given by (31). Right: Values of the curvature based test statistics $\frac{G}{M^2}$, defined in (32) plotted against mean curvature *M* for the same data as in the left part.

Figure 9 - Pair Plots

Pair plots of metabolite intensities, the axes are labeled with the respective metabolite indices. Triangles mark data points from the *Col*10 x *Col*10 crossing, circles from the C24 x *Col*10 crossing. The different colors indicate the individual biological replicates, in addition the more "earthly" colors are redundantly attributed to the C24 x *Col*10 crossing. A strong correlation (up to 0.98) can be seen in the technical replicates. The diagrams also show a strong relation between technical and biological variabilities.

Figure 10 - Test: Entropy

Entropy of the observed data (black spot) and 10 draws from the hypotheses plane (green spots), line (red circles) and point (blue triangles). The different metabolite triplets under investigation are indexed on the abscissa.

Figure 11 - Test: Polynomial Measure of Concentration Polynomial measure of concentration of the observed data (black spot) and 10 draws from the hypotheses plane (green spots), line (red circles) and point (blue triangles). The different metabolite triplets under investigation are indexed on the abscissa.

Figure 12 - Test: Curvature based Test Statistics Curvature based Test Statistics of the observed data (black spot) and 10 draws from the hypotheses plane (green spots), line (red circles) and point (blue triangles). The different metabolite triplets under investigation are indexed on the abscissa.

Figure 13 - Metabolite Triplets

Part a.) Metabolite intensities of triplet #7, consisting of metabolites #278, #523 and #715, showing a pattern that visually resembles a plane in 3-space. Part b.) Metabolite intensities of triplet #14, consisting of metabolites #322, #380 and #290. The data are distributed along a line through the origin in 3-space

Figure 14 - Posteriors

Posteriors for the triplets in Figure 13. Part a.) Posterior of triplet #7: The beta marginal as well as the map for the sphere part indicate an uni-modal posterior that is characteristic for the presence of a single plane. Part b.) Posterior of triplet #14: The beta marginal reaches its maximum at β=0 while in the sphere part map a ringlike structure can be seen, both indications compatible with the "line"-hypothesis.

Figure 15 - Triplet Selection

Pair plots of selected metabolites. From the total of 763 metabolites first the 5% quantile of the ratio between biological variability and technical precision was taken. In a second step from this high precision metabolites those ones were further selected, and plotted here, whose median of the correlation coefficient, taken over the remaining metabolites, was in the 5% to 30% percentile. Finally metabolites #71, #693 and #713 were chosen for the classification. The crossings are marked by red for *Col*10 x *Col*10, green for C24x C24, blue for *Col10* x C24 and yellow for C24 x *Col10*.

Figure 16 - Classification in 3-space

Screen shot of an interactive perspective view of the classification described in section 2.9.3. The data of the metabolite intensities are depicted by small balls, colored in red for the *Col*10 x *Col*10, in green for the C24 × C24 and blue for the *Col*10 x C24 and C24 x *Col*10 group. Misclassified values were depicted in white. The indicator planes resulting from the Monte Carlo optimization process, and their descriptor vectors, were drawn in the colors of their groups. The axis system is displayed in white and labeled with the metabolite numbers.

<u>The Set of Planes</u>

Consider the 3-dimensional Euclidean space $R^3$. A 2-plane is a two dimensional affine subspace. The set of all 2-planes is a 3 dimensional manifold. Unfortunately, there is no global parameterization of this space. However, there is a parameterization that is good for all practical purposes.

Consider now a unit-vector $\vec{n}$ and a real number β≥0. The set of points $\vec{x}$ that have in the direction $\vec{n}$ the distance β is a 2 dimensional plane. In formulas one may write (see Figure 1):

$$E = \left\{ \vec{x} \in R^3 \middle| (\vec{x} \cdot \vec{n} - \beta) = 0 \right\} \qquad (1)$$

Vice versa all planes that do not contain the origin can be written uniquely in that way. The planes that go through the origin can still be written in that way, however, the representation is not unique anymore, since $\vec{n}$ and $-\vec{n}$ give rise to the same plane. Nevertheless one identifies

$$E \leftrightarrow (\beta, \vec{n}) \ .$$

The unit vector itself may be written in polar coordinates using two angles θ∈[0,π] and ϕ∈[0,π]. One then may write according to Bronstein I, Semendjajew K, ea: Taschenbuch der Mathematik. Thun und Frankfurt am Main: Verlag Harri Deutsch 2001,

$$\vec{n} = \begin{pmatrix} \sin[\theta] \cdot \cos[\phi] \\ \sin[\theta] . \sin[\phi] \\ \cos[\theta] \end{pmatrix} \quad (2)$$

where the usual care has to be taken for θ=0 and θ=π since there the parameterization degenerates. In that way one obtains a parameterization

$$E \leftrightarrow (\beta, \theta, \phi) \in [0, \infty) \times [0, \pi] \times [0, 2\pi)$$

where care has to taken at the points θ∈{0,π} and β=0.

Invariant Measure

Since it is the aim to perform Bayesian analysis on the set of possible planes, which is a manifold as described above, one has to determine a measure on it.

One needs this measure for integrating over the posterior (if one, for example wants to calculate measures of concentration like entropy) and as a non informative prior. The complete absence of information should be invariant under the natural coordinate changes: no information is gained by translating the whole space or rotating it. Therefore the measure that one is looking for should be invariant under the group generated by rotations and translations (i.e. the Euclidean group). One denotes by

$$\vec{x}' = \mathbf{R}\vec{x} \quad \text{with} \quad \mathbf{R}^{-1} = \mathbf{R}^{T} \quad (3)$$

the rotations and by

$$\vec{x}' = \vec{x} + \vec{a} \quad (4)$$

the translations of points in three dimensional space $R^3$.

In the present case one can show that there is an up to a multiplicative constant unique measure that satisfies these invariance properties. The proof is following the method given in Jaynes E: The Well-Posed Problem. Foundations of Physics 1973, 3(4):477-491; Jaynes E, Bretthorst G: Probability Theory. The Logic of Science: Theory and Elementary Applications Vol. 1. Cambridge: Cambridge University Press 2003. In the parameterization given by (2) this invariant measure in the space of planes $d\mu(E)$ can be expressed as

$$d\mu(E) = const \cdot \sin[\theta] d\theta \, d\phi \, d\beta \; . \quad (5)$$

Note that the points, where the parameterization becomes singular only have measure 0. So the parameterization is fine for any distribution of planes which does not have a point mass at theses singular values.

Error Model

Until now planes are just geometrical entities but not models in the statistical sense. To use them as a tool for (Bayesian) statistical analysis one augments them by an error model. More precisely, one has to make an assumption that if a given quantity in reality has the value $\vec{y}$ with a given probability $p(\vec{x}|\vec{y})$ one observes the value $\vec{x}$ instead. This conditional probability then is the error model.

In the context of the present description of the invention one uses a multivariate Gaussian error model with covariance matrix Σ and zero systematical error:

$$p(\vec{x}|\vec{y}) \sim \exp\left[-\frac{1}{2}(\vec{y}-\vec{x})^T \Sigma^{-1}(\vec{y}-\vec{x})\right] \qquad (6)$$

The error model gives us the probability that a single data point $\vec{x}$ results from a single, true point $\vec{y}$ . .

One now wants to compute the probability distributions of a single observation that comes from some location on a fixed plane *E.* For this, one needs to specify the distribution of points on the plane $dp(\vec{y})$ on the plane. The distribution of a single observation coming from the plane *E* is then computed as

$$p(\vec{x}|E) = \int_E p(\vec{x}|\vec{y})dp(\vec{y}) \qquad (7)$$

Since one does not have any a priori information about the localization of the points $\vec{y}$ on the plane one assumes an uniform and hence improper distribution. Thus $dp(\vec{y})$ is simply a Euclidean surface measure. Remembering that the plane *E* itself is parameterized by E=*E*( $\vec{n}$,β) one arrives at a result analogous to eqn. (14) in Kose et al. for the Likelihood of plane *E*=*E*(*n*,β):

$$L(\vec{n},\beta|\vec{x}) \sim \exp\left[-\frac{1}{2}\frac{(\vec{x}\cdot\vec{n}-\beta)^2}{\vec{n}^T\Sigma\vec{n}}\right] \qquad (8)$$

For fixed *E* as a function of $\vec{x}$ , this density is an improper Gaussian distribution with degenerated covariance matrix. It shows that the probability distribution that a given point $\vec{x}$ stems from a certain plane *E* depends only on its normal distance. The dependency on the plane itself is only through its normal vector $\vec{n}$ and distance to the origin of β.

By the theorem of Bayes and the assumption of a uniform prior with respect to the invariant measure (5) this is proportional to the posterior of a plane *E* given a single data point $\vec{x}$ :

$$p(E|\vec{x}) = p(\vec{x})^{-1} p(\vec{x}|E)p(E) \sim p(\vec{x}|E) \qquad (9)$$

To get an intuition how this function looks like, one draws a figure that shows which planes are able to "explain" a given data point perfectly. This means one asks for the subset of planes that have zero distance to the data point.

The distance of a point $\vec{x}$ to a plane $E=E(\vec{n},\beta)$ computes to $\left|\vec{x}\cdot\vec{n}-\beta\right|$. If we set it to zero we can see that the vector $\beta\cdot\vec{n}$, called the *descriptor* of the plane, has to form an rectangular triangle with $\vec{x}$ as hypotenuse. From Thales' theorem one sees that the set of descriptors belonging to planes that explain a given data point is a sphere through the data point and the origin of the coordinate system. Figure 2 shows this in two dimensions, rotating the drawing around $\vec{x}$ *x* gives the sphere.

From this considerations one sees that the probability density of a single data point attains a constant, maximal value on the Thales' sphere and decreases in accordance to the covariance parameters Σ of the error model.

Robust Posterior

After obtaining the expression (8) for the probability of a given plane $E=E(\vec{n},\beta)$ with respect to a single data point $\vec{x}$ one now has to find an expression for the posterior $p(E|\{\vec{x}_i\},...)$ of a plane $E$ with respect to a set of data points $\{\vec{x}_i\}$ where $i\in\{1,2,...,N\}$.

A principle desideratum in data analysis is *robustness.* This means that the conclusions drawn from a data set do not change strongly if one or even few of the data points, called outliers, result from processes that have nothing to do with the phenomenon generating the variability under investigation. Examples are mistakes in the measurements or sample handling or genetic or environmental deviations concerning single biological organisms in the sample. An example of a method of analysis that is not robust in this sense is Pearson's coefficient of correlation for small sample sizes, since a single outlying data point sufficiently distant from the meaningful rest of the population can twist the result away from the biologically correct number.

To arrive at an intrinsically robust formula for the posterior one follows the concept of Kose et al. and assumes implicitly that with a given plausibility any of the values in a set of data points $\{\vec{x}_i\}$ *may be* an outlier. For the posterior density $\vec{p}(n,\beta|\{\vec{x}_i\},c)$ (Kose et al.) arrive at following formula:

$$\ln\left[p\left(\vec{n},\beta\middle|\{\vec{x}_i\},c\right)\right]\sim\sum_{i=1}^{N}\ln\left[p\left(\vec{n},\beta\middle|\vec{x}_i\right)+c\right] \qquad (10)$$

$$=\sum_{i=1}^{N}\ln\left[\exp\left[-\frac{1}{2}\frac{\left(\vec{x}_i\cdot\vec{n}-\beta\right)^2}{\vec{n}^T\sum_i\vec{n}}\right]+c\right] \qquad (11)$$

Therein c, called the "decoupling term", reflects the prior that a given data point is in fact an out lier. It ensures the robustness of the formula since vanishing of a single term $\vec{p}(n,\beta|\vec{x}_i)$ does, for values of $c\approx 1$, not result in infinitely negative value of the logarithm, and so limits the maximum influence of a single data point on the sum to $\ln[c]$. In the subsequent analyzes one sets the numerical value to unity $c=1$, in accordance with Kose et al.

It was shown by Kose et al. that this formulation of the posterior (10) can be utilized to mixtures of data consisting of multiple linear relations, given approximate values of c. In a Bayesian sense this can be understood in the following way: The summation of single point posterior $(\vec{n},\beta|\vec{x}_i)$ and decoupling term c in (10) describes a situation in which the individual data point can either be attributed to the plane $\vec{E}(n)$ or the completely uninformative alternative represented by an constant, improper likelihood. The possibility of investigating multiple linear relationships now corresponds to the assumption, that any of the other possible planes might be "absorbed" in this isotropic alternative.

Discretization

To represent numerically and visualize the posterior one has to find a discretization of the set of planes under consideration.

For any data set one can, before the computation of the posterior, see that the possible distance to the origin is limited to some interval $\beta\in[\beta_{min};\beta_{max}]$. In this interval one discretizes β by a set of equidistant values $\{\beta_{min},.....\beta_b,....,\beta_{max}\}$.

The discretization of the sphere part of the posterior, for every of the discretized values of $\beta_b$, is taken over from Freeden W, Gervens T, Schreiner M: Constructive Approximation on the Sphere: With Applications to Geomathematics. Oxford: Claredon Press 1998.

The polar angle $\theta$ is represented by a set of $\gamma$ equidistant points $\{\theta_0,...,\theta_i,...\theta_\gamma\}$ with $\theta_0=0$(*north pole*) and $\theta_\gamma=\pi$ (*south pole*).

The azimuthal angle $\phi$ is also discretized by an equidistant lattice $\{\phi_{i,0},...,\phi_{i,j},...,\phi_{i,\gamma i}\}$. Here the number of points of discretization $\gamma_i$ depends on $\theta_i$ in a way ensuring that the points of discretization $(\theta_i,\phi_{i,j})$ are uniformly spread over the surface of the sphere , cf. Freeden et al.:

$$\Theta_0 = 0 \quad \phi_{0,1} = 0 \ north\ pole \qquad (12)$$

$$\Delta\Theta = \frac{\pi}{\gamma}\ with\ \gamma \in N \qquad (13)$$

$$\Theta_i = i \cdot \Delta\Theta\ with\ i \in \{1, 2, ..., \gamma - 1\} \qquad (14)$$

$$\gamma_i = \frac{2\pi}{\arccos\left[\left(\cos[\Delta\Theta] - \cos^2[\Theta_i]\right) / \sin^2[\Theta_i]\right]} \qquad (15)$$

$$\phi_{i,j} = \left(j - \frac{1}{2}\right)\left(\frac{2\pi}{\gamma_i}\right)\ with\ \ j \in \{1, 2, ..., \gamma_i\} \qquad (16)$$

$$\Theta_\gamma = \pi \quad \phi_{\gamma,1} = 0\ \ south\ pole \qquad (17)$$

The angular resolution depends on the integer parameter $\gamma$ which gives the number of lattice points in $\Theta$ direction, so before the calculation of the posterior, after specifying the data and the parameters of the error model, one has to find an numerically adequate value for it. To obtain a practical estimate one looks again at the posterior of a single data point (8):

$$p(\vec{n}, \beta|\vec{x}) \sim \exp\left[-\frac{1}{2}\frac{(\vec{x}\cdot\vec{n} - \beta)^2}{\vec{n}^T \Sigma \vec{n}}\right]$$

One considers the generic case of a data point $\vec{x} = x \cdot \vec{n}_z$ situated at distance $|\vec{x}|$ in "north" direction $\vec{n}_z = \vec{n}(\theta = 0, \phi = 0)$. The error model is assumed to be isotropic with $\Sigma = \sigma^2 \cdot I$.

Since here the scalar product $n \cdot x = |x| \cdot \cos\theta$ does not depend on $\phi$ the posterior is a function of $\theta$ and $\beta$ alone. One uses a saddle point approximation around the maximum value $\theta^* = \arccos[\beta / |\vec{x}|]$, $\beta^* = |\vec{x}|$ and finds that it is approximately proportional to:

$$p(\theta, \phi, \beta^*) \sim \exp\left[ -\frac{1}{2} \frac{(\beta^*)^2 \sin^2 \theta^* (\theta - \theta^*)^2}{\sigma^2} \right] \quad (18)$$

The standard deviation in angular direction $\sigma_\theta$ is:

$$\sigma_\theta = \frac{\sigma}{|\vec{x}|} \cdot \frac{1}{\sqrt{1 - \beta / |\vec{x}|}} \quad (19)$$

It has its minimum at $\sigma_\theta = \sigma / |\vec{x}|$, so the angular standard deviation depends on the relative error $\sigma_{rel} \equiv \sigma / |\vec{x}|$ of the data.

From identical arguments one sees that the standard deviation in $\beta$ direction $\sigma_\beta = \sigma$, and hence depends on the absolute error $\sigma_{abs} \equiv \sigma$.

One uses this insight and to the number of points $\gamma$ in $\theta$ direction to

$$\gamma = ceiling\left\{ \frac{res_\theta \cdot \pi}{\sigma_{rel}} \right\} + 1 \quad (20)$$

and the spacing of the points in $\beta$ direction to:

$$\Delta\beta = \frac{\sigma_{abs}}{res_\beta} \quad (21)$$

Herein $res_\theta$ and $res_\beta$ are the precision of the discretization. In the actual choice of their values a tradeoff between runtime of the calculation and precision has to be kept in mind. Numerical investigations showed that all ready a value of $res_\theta = res_\beta = 3.6$ gave very accurate results.

In dealing with different, anisotropic estimates for the standard deviation of the error one calculates the relative and absolute error in the data for each single point, using the eigenvalues of the covariance matrix $\Sigma_i$, and took the maximum of the resolution.

Visualization

Mathematically the posterior $p(\vec{n},\beta|\{\vec{x}_i\},c)$ is a probability density over the distance β and the unit vector $\vec{n}$. For visualization of its sphere part, this is the dependence on the unit vector $\vec{n}$ given a fixed value of the radius β, in analogy to geographical *map projections* one uses an (almost) area conserving mapping of the coordinates θ and ϕ onto the screen. For simplicity of the formula one uses the Kavrayskiy VII projection (Wikipedia, English: "Kavrayskiy VII projection" [http://en.wikipedia.org/wiki/Kavrayskiy_VII]). The logarithm of the posterior is then displayed on this map by a colour coding.

To inspect the dependence of the posterior on the radius one integrates out the angular dependence and obtains the *marginal distribution* over β, the "β-marginal":

$$p(\beta|\{\vec{x}_i\},c)=\int_0^{2\pi}\int_0^{\pi} p(\phi,\theta,\beta|\{\vec{x}_i\},c)\sin[\theta]d\theta\, d\phi \qquad (22)$$

In a software prototype according to the method of the present invention the inventors realized the simultaneous visualization of sphere part and β-marginal by arranging the color coded map of the sphere-part for a given value of β above a white bar-chart of the logarithm of the β-marginal (22). To indicate the actual value of β the respective bar in the bar chart is marked in red. One includes two forms of interactivity: First one makes it possible to explore the values of β by mouse clicking on the respective position of the β-marginal bar. Second one allows the user to select planes by clicking on the sphere part map. The selected planes are then stored in a list and displayed as blue numbers in the diagram.

In Figure 3 as an example the posterior of the Thales sphere resulting from a single data point is visualized, as described in section "Error Model". In Figure 3.a the sphere part of zero radius β = 0, which is a great circle along the "Greenwich meridian" of the map, can be seen. Figure 3.b shows the case for an intermediate value of β lying in between the data points $\vec{x}$ and the origin of the coordinate system. The intersection between the cone defined by the Thales sphere and the space of unit normals $\vec{n}$, which is the sphere part, now forms a circle. If β equals the distance of point $\vec{x}$ to the origin, as in Figure 3.c, this circle degenerates to a point that because of the error model is visible as a round blob.

One of the main advantages of the robust posterior is the possibility of investigating multiple linear relationships resulting in multi-modal posteriors. To demonstrate this in Figure 4 one looks at a situation in which 100 data points are scattered on one plane (green points) and 50 data points are scattered on an other. In Figures 5.a and 5.b one sees the posterior at the respective values of β. The bimodality is clearly visible from the betamarginal and the sphere part.

Posterior Based Test Statistics

In addition to the visual inspection of the calculated posterior the inventors developed a statistical test for the presence or absence of plane related structures in the data. Therefore the inventors supplemented the Bayesian approach with conventional frequentist test statistics (Rinne H: Taschenbuch der Statistik 2. überarbeitete und erweiterte Auflage. Thun und Frankfurt am Main: Verlag Harri Deutsch 1997). The idea behind this is the intuition that test functions should get advantageous properties if they are somehow related to the hypotheses to be tested for. The inventors did not rely on a fully Bayesian treatment of the test situation since the computation of the marginal posterior likelihoods (Bayes factors) of the model classes acting as alternative hypotheses would involve their fully Bayesian treatment. The inventors defined a posterior based test function as functional of the posterior:

$$T(\{\vec{x}_i\})=T\{p(\vec{n},\beta|\{\vec{x}_i\},c)\} \qquad (23)$$

The statistical test is then constructed in the common way: One states a statistical *null hypothesis* $H_0$ as well as an *alternative hypothesis* $H_1$ by defining a probability densities for the data

$$p(\{x_i\}|H_0) \text{ and } p(\{x_i\}|H_1) \quad (24)$$

which, on a geometrical level, are related to distinct plane related structures. Then one calculates, by Monte Carlo methods, the distributions of the test function:

$$p(T|H_0) \text{ and } p(T|H_1) \qquad (25)$$

After determining the empirical value of the test function $T^* = T(\{\vec{x}_i\})$ from the experimental data $\{\vec{x}_i\}$ one compares it to the distributions and draws conclusions on the validity of the hypotheses, i.e. the presence and absence of the underlying plane related structures (cf. Rinne).

Concentration Based Test Statistics

Two different approaches for constructing the test function are presented. The first one is based on measures of concentration of the posterior like the *entropy*

$$S(\{\vec{x}_i\}) = \int p(E|\{\vec{x}_i\}) \cdot \ln p(E|\{\vec{x}_i\}) \; d\mu(E) \qquad (26)$$

or the *polynomial measure of concentration:*

$$PMOC(\{\vec{x}_i\}) = \int p(E|\vec{x}_i)^q \; d\mu(E) \qquad (27)$$

Herein $q$ is the exponent of the polynomial, that was constantly set to $q$ = 0.5.

The capability of these functions to distinguish between certain plane related structures by defining the two hypotheses was investigated:

$H_0$: [Null Hypothesis] Data points are first casted isotropically on a square plane of size 20 × 20 and afterwards are subjected to a 3-dimensional Gaussian error with standard deviation σ = 1

$H_1$: [Alternative Hypothesis] Data points are scat- tered isotropically in a cube of size 20 × 20 × 20

Then repeatedly samples of $N$ data points are drawn and, assuming an isotropic error with standard deviation σ = 1, one calculates the test functions, in this way obtaining a sample from the test distribution (25). As can be seen from Figure 6 and Figure 7 the entropy as well as the polynomial measure of concentration were indeed able to separate the two hypotheses under the given conditions.

Curvature Based Test Statistics

The second approach for constructing the test function is based on measures of the local curvature of the sphere part of the posterior, calculated in the immediate neighbourhood of its global maximum.

To define the invariant local measures of curvature, the *mean curvature M* and the *Gaussian curvature G*, one uses the sphere part of the posterior that remains if one fixes β to the value β* where the posterior attains its global maximum:

$$p\left(\vec{n},\beta^{*}\middle|\{\vec{x}_i\},c\right)\ \text{with}\ \left(\vec{n}^{*},\beta^{*}\right)=\arg\max_{\vec{n},\beta}\left(p\left(\vec{n},\beta\middle|\{\vec{x}_i\},c\right)\right)\qquad(28)$$

After parameterization (2) this is a function of the two angles θ and ϕ. One concentrates its interest into the immediate neighbourhood around the global maximum of the posterior, that occurs at θ* and ϕ*. From the values of (28) in this neighbourhood one can now compute the local values of the main radii of curvature $R_1$ and $R_2$ at the point (θ*, θ*,β*) (cf. Bronstein et al.).

From the main radii of curvature one obtains the invari*ants* of the curvature tensor, the mean curvature

$$M=\frac{1}{2}\left(\frac{1}{R_1}+\frac{1}{R_2}\right)\qquad(29)$$

and the Gaussian curvature:

$$G=\frac{1}{R_1\cdot R_2}\qquad(30)$$

The definition of the test statistic itself is based on the observation that, in case the data are in fact straying around a plane, the sphere part (28) forms a circular "blob" around the global maximum. This circular symmetry implies that the main radii of curvature are equal $R_1$=$R_2$, which leads to following simple relationship between mean and Gaussian curvature:

$$G=M^2\qquad(31)$$

In the left part of Figure 8 this is illustrated by plotting the Gaussian against the mean curvature for data points sampled from different, synthetically generated hypotheses, namely data stray around a plane, a line or a point. The exact mathematical definition of the hypotheses is given later. One can see, that the values for plane generated data points approximately lie on the curve predicted by (31), while for the two remaining hypotheses the Gaussian curvature vanishes.

This leads to the definition of a *curvature based test function* by:

$$T_c\left(\{\vec{x}_i\}\right)=\frac{G}{M^2}\qquad(32)$$

By definition this lies in the [0,1] interval. In the right part of Figure 8 one plots values for the given data points and notices its discriminating power under the given circumstances.

Supervised Learning

The notion of plane posteriors (8) gives the opportunity to construct an algorithm for classifying data with respect to inherent plane-like structures.

Considerations start with a set of data points ($\vec{x}_i$), with adequate estimations of the observational error {$\Sigma_i$}, joint together into a number of groups

$$G_k = \{\vec{x}_i\}_k \qquad (33)$$

which are assumed to be related to plane-like structures inherent in the data.

In general an algorithm for supervised learning uses this information to obtain an indicator function that attributes an additionally given data point to one of those groups (Hastie T, Tibshirani R, Friedman J: The Elements of Statistical Learning. New York: Springer 2001).

In the algorithm it is proposed here this indicator function is realized by the specification of an indicator plane $E_k$ for each of the groups $G_k$. The indicator function is defined as a mapping of the point $x$ to be classified, given its proper error estimation $\Sigma$, to that group, whose indicator plane results in the maximum posterior value with respect to $\vec{x}$:

$$I(\vec{x}) : \vec{x} \to \arg\max_{E_k} \{p(\vec{x}|E_k)\} \qquad (34)$$

The indicator planes are generated by an optimization process based on the Metropolis algorithm for drawing a sample from a Boltzmann type distribution (cf. Gilks W, Richardson S, Spiegelhalter D: Markov Chain Monte Carlo in Practice. New York: Chappman & Hall 1996). Therefore for each group $G_k$ an energy-like scoring function $H_k\{E_m\}$ is defined, that measures the appropriateness of a given candidate plane $E_m$ for positively discriminating that specific specific group $G_k$:

$$H_k(E_m) = \sum_l \alpha_{l,m}\, p(E_m|\{\vec{x}\}_l) \qquad (35)$$

Here $\{\vec{x}_i\}_l$ is the set of data points belonging to any of the groups $G_l$ with $l$=1,2,...,$k$,... and $\alpha_{l,m}$ is a set of properly chosen weights that penalize posterior based distance to the target group $G_k$ as well as closeness to all the other groups. The most basic choice for the weights, that was used, is:

$$\alpha_{l,m} = \begin{Bmatrix} +1 \ for \ l = m \\ -1 \ for \ l \neq m \end{Bmatrix} \qquad (36)$$

Then a classical Metropolis process is executed (Gilks et al.) that generates a sample of planes $\{E_m^1, ..., E_m^t, ..., E_m^{t_{max}}\}$ with $t$=1,...$t_{max}$ out of the Boltzmann distribution:

$$p(E_m) \sim \exp[H_k(E_m)] \qquad (37)$$

Finally for the group $G_k$ the indicator plane $E_k$ is selected from the output $\{E_m^t\}$ of the Monte Carlo process by taking the argument at the maximum of the group specific scoring (35):

$$E_k = \arg\max_{\{E_m^t\}} \{H_k(E_m)\} \qquad (38)$$

The method of the present invention enriches the toolbox of systems biology with the novel method, called ExPlanes, for the exploration of ternary relationships in profile data that combines following features: Invariance against rotations and consequently relabeling of the axes, which makes it especially suitable in cases where no a-priori ordering of the variables can be assumed. In this it differs from usual regression methods. Possibility to accommodate robustness against outliers and incorporation of an individual error estimate for each data point. The possibility of investigating multiple linear relationships. Different test-statistics enable the detection of a variety of plane related ternary structures, to be defined by appropriate statistical hypotheses. Supervised learning of classification problems that give further structural insight is possible with respect to appropriate plane related structures in the data. The method also gives an intuitive way to visualize the Bayesian posterior in plane space for exploratory purposes. The description demonstrated all of these aspects of the method according to the invention for an real world example of metabolic data.

The method of the present invention is especially useful for cases, where a learning problem is suspected to show inherent plane related structures, and as an tool for exploratory data analysis and network generation.

**Example:**

Plant Metabolite Data

The inventors applied the method according to the invention described above to triplets of metabolic data, generated in an experiment published earlier by Scholz et al. We first describe the experiment and the data, then the inventors applied the methods according to the present invention described in section "Posterior Based Test Statistics" to extract the presence of plane-like structures and visualize them exemplarily. Finally the inventors use the method for supervised learning for classifying the data according to their genetic crossings from a triplet of individual metabolites.

Description of Experiment and Data

In the experiment parent plants from the two *Arabidopsis* thaliana inbred lines *Col*10 and C24 were mated, resulting in four possible combinations of parent genotypes. For each of this crossings three F1 hybrids were grown (so called *biological replicates),* after harvesting and preparation for approximately eight samples (*technical replicates*) the intensities of 736 metabolites, identified by the mass to charge ratio (m/z), were measured. Since no identification of the chemical species was done in the mass- spectrometric data, the metabolites were identified by arbitrary indices. In Table 1 the peak position of the mass to charge ratio (m/z) of selected metabolites of interest is given (Scholz et al.).

Table 1: Peak mass to charge ratio (m/z) of selected metabolites [Scholz et al.].

| **Metabolite** | **(m/z)** |
|---|---|
| # 71 | 106.99 |
| # 278 | 240.00 |
| # 290 | 733.10 |
| # 322 | 959.16 |
| # 380 | 960.16 |
| # 523 | 861.66 |
| # 693 | 872.19 |
| # 713 | 840.18 |
| # 715 | 938.18 |

Before one starts the Bayesian analysis itself, one explores the data by investigating different measures for technical and biological variability:

A quantitative description of the precision of the data is the *technical variability* defined as the standard deviation of technical replicates made from one biological replicate.

One defines the *estimated biological* value as the mean value of the technical replicates made from one biological replicate, it serves as an estimator for the true value of the metabolite concentration in the plant.

For a given set of plants, not necessarily being biological replicates of a single crossing, one defines the bio*logical variability* as the standard deviation of their estimated biological values.

Based on these quantities one chooses a sample combined out of the biological replicates of the C24 × *Col*10 and the C24 × C24 crossings for further investigation, because it showed the most promising ratio of (large) biological to (small) technical variability.

One sorts the metabolites according to the ratio of biological to technical variability and selected the first six metabolites, #278, #322, #523, #380, #290 and #715, from that list. Those we combined in all possible, non redundant ways to triplets, which were then the 3-dimensional input to Bayesian method described above.

Testing Plane Related Hypotheses

The empirical covariance matrix of the technical replicates was used as Σ in the Gaussian error model (6) for each data point resulting from the underlying biological replicate.

The necessity for using a correlated error model is evident if one looks at the *pair plots* of the metabolite intensities as well as the numerical values of the covariances, as exemplified in Figure 9 for metabolite pairs #380 × #322 and #523 × #278. This a very strong correlation in the technical replicates (up to 0.98). The diagrams also indicate that the technical and biological variabilities are related, which can be interpreted in the sense that the same biochemical mechanisms acting in the intact plants are responsible for the variability occurring during preparation and measurement.

Three test hypotheses were defined, qualitatively related to the geometrical structures point, line and plane. For each metabolite triplet under investigation the parameters of the probability distribution forming the statistical hypotheses was then set depending on numerical values characterizing the data.

The centre of the data was defined as the median of the biological estimated values of the biological replicates under investigation. The mean technical covariance was defined as the average of the covariance matrices of the technical replicates over the biological replicates.

The hypotheses were defined algorithmically by:

Point Gaussian distribution with mean technical co- variance around the centre of the data.

Line Draw a line from the origin through the centre of the data. Distribute the points on that line with a Gaussian distribution of zero mean and variance equal to the sum of the squared biologi- cal variability of the metabolites involved. Add centred Gaussian noise with technical covariance.

Plane Scatter the points on a plane trough the centre of the data, employing a radial symmetrical Gaus- sian distribution with a variance equal to the mean of the squared biological variability of the metabolites involved. Add centred Gaussian noise with technical covariance.

From these distributions inventors drew 10 sample points and calculated entropy (26), polynomial measure of concentration (27) and curvature based test statistics (32). In that way the inventors obtained small size samples of these quantities given the test hypotheses that the inventors compared with the data measured in the experiment.

Figure 10 to 12 show these results. On the abscissa the metabolite triplet under consideration is indexed, on the ordinate the value of the test statistics is displayed.

Green, blue and red points mark the values generated by the test hypotheses plane, point and line, while the value originating from the experimental data are indicated in black.

For the entropy Figure 10 and the polynomial measure of concentration Figure 11, the inventors notice that they are in principle able to separate the three different hypotheses, in contrast to the curvature based test function Figure 12, that cannot distinguish between points and lines but more distinctively highlights true plane like structures.

For the majority of the triplets the results suggest that they can be described by the "line" hypothesis.

Further visual inspection of the data supported this findings as can be seen in Figure 13 showing typical patterns for "plane-" (Triplet #7, part a.) ) and "line-" (Triplet #14, part b.) ) data that visually convincing resemble the geometrical meaning of these hypotheses. However care must be taken in comparing the visual appearance of the data to the statistical hypotheses which are based on the error model and not on the distance in 3-space directly. Therefore the inventors used the methodology that incorporates this information to visualize the posterior.

Figure 14 shows screen shots of the interactive visualization of the posterior at the respective maximal points of the β-marginal. In part a.) the posterior for triplet #7 indicates an uni-modal posterior that is characteristic for the presence of a single plane. In part b.) the beta marginal for triplet #14 reaches its maximum at β = 0 while in the sphere part a ring like structure can be seen, both indications compatible with the "line"-hypothesis; also compare Figures. 10, 11 and 12

Supervised Learning

The data set under investigation is labeled for the four different possible crossings resulting from the possibilities of selecting the parents from two different inbred strains. Characterization with respect to this property was the original aim of the paper (cf. Scholz et al.). Here the method was used given in section "Supervised Learning" to classify directly with respect to a manually selected triplet of metabolites.

The triplet used for classification was selected according to a procedure that was intended to focus on metabolites first with high ratio between biological variability and technical precision and second of low, but not to low, pair correlations:

- The ratio between biological variability and technical precision was calculated.
- A set of *high precision metabolites* was defined as the 5% quantile of this ratio.
- For these metabolites the correlation matrix was calculated.
- For every metabolite the median of the correlation was taken over the remaining metabolites.
- From the sample of this median values the percentile between 5% and 30% was selected.
- An appropriately colored pair plot of this metabolites was generated (Figure 15).
- Metabolites #71, #693 and #713 were finally selected.

Metabolites #693 and #713 were selected because of their good separation between the *Col*10 × *Col*10 and C24 × C24 crossing and #71 for the separation of *Col*10 × C24 and C24 × *Col*10 from the others.

The 92 data points for this metabolite triplet were classified with the method defined in section "Supervised Learning" with respect to three groups. The first group $G_1$ contained the *Col*10 × *Col*10 crossing, the second group $G_2$ the C24 × C24 crossing while crossings *Col*10 × C24 and C24 × *Col*10 were put together in a third group $G_3$. The inventors believed those not suitable for further separation, because there was no apparent biological difference in the data between male or female parent coming from the different inbred lines.

With $\alpha_{l,m}$ given by (36) the Metropolis chain was run for $t_{\max}$ = 500 iterations with a uniform trial distribution in angular and radial direction, and resulted in a correct classification of 90 out of the 92 data points. In Figure 16 a colored view of this classification is given.

To asses the rate of misclassification s of the method in the given data set a *leave some out* error estimation was done. The inventors selected at random 92 samples of 82 out of the 92 original data points, according to a loss of about 10% of the data, as training sets. For each of this training sets the inventors learned the indicator planes and classified the remaining data points, called the test set, as described above. In 21 of the 92 cases one data point, in one case two data points were misclassified, the other 70 cases were classified correctly.

In addition the inventors performed a *leave one out* error estimation with 92 training sets of 91 points. A misclassification of the remaining point occurred in 4 of the 92 cases, corresponding to a leave one out error of 4.35%.

**Claims**

1. Method for investigating ternary relationships in a set of electronically storable data, wherein planes are investigated, by

    a) determining flat prior from a rotational and translational invariance, and then
    b) determining the Bayesian posterior in the space of all possible 2 planes in 3 space, and
    c) visualisation of the posterior function.

2. Method according to claim 1, **characterized in that** the Bayesian posterior is based on an error model, which is a mixture of a Gaussian and an uniform distribution.

**3.** Method according to one of the preceding claims, **characterized in that** for the visualisation the Bayesian posterior for all directions of the normal vector of the planes is displayed for a fixed beta value and that additionally the beta marginal of the Bayesian posterior is displayed.

**4.** Method according to claim 3, **characterized in that** the given β-value is mapped in the presentation of the β-marginal.

**5.** Method according to claim 4, **characterized in that** the β-value is varied in the presentation of the β-marginal, whereby the presentation of the Bayesian posterior is visualized for the varied β-value.

**6.** Method according to one of the preceding claims, **characterized in that** a further step is performed after step b) and before step c), wherein a set of statistical tests is defined, the said tests being based on a family of test statistics that are defined as functional of the posterior, whereby specific hypotheses about the structure being present in the measured data are made detectable.

**7.** Method according to claim 6, **characterized in that** the test functions are selected from curvature based test statistics and concentration based test statistics like entropy or polynomial measures of concentration

**8.** Method according to claim 6 or 7, **characterized in that** the result of at least one of the tests is outputted.

*Fig. 1*

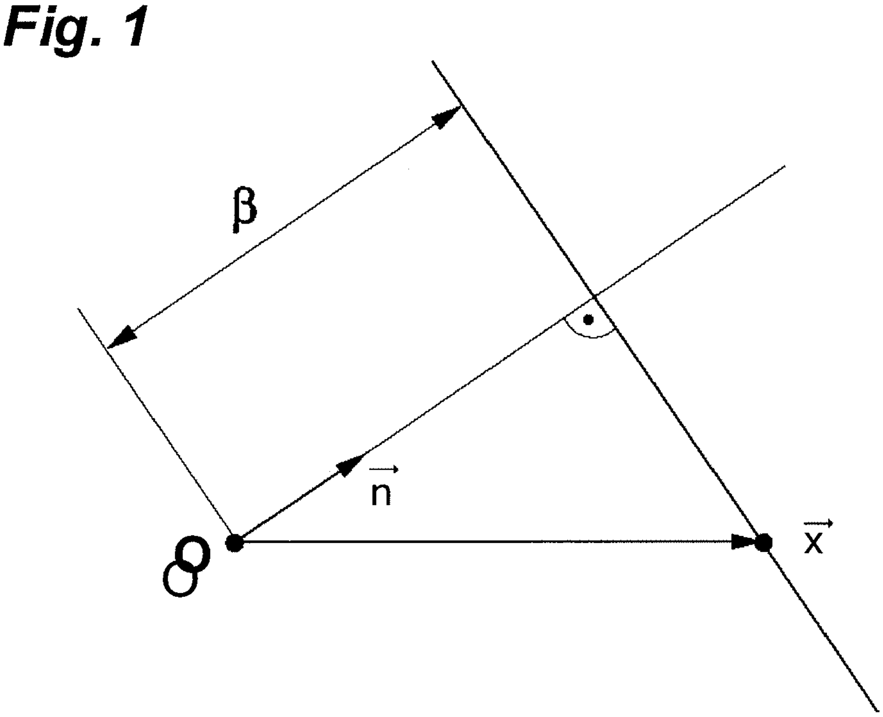


*Fig. 2*

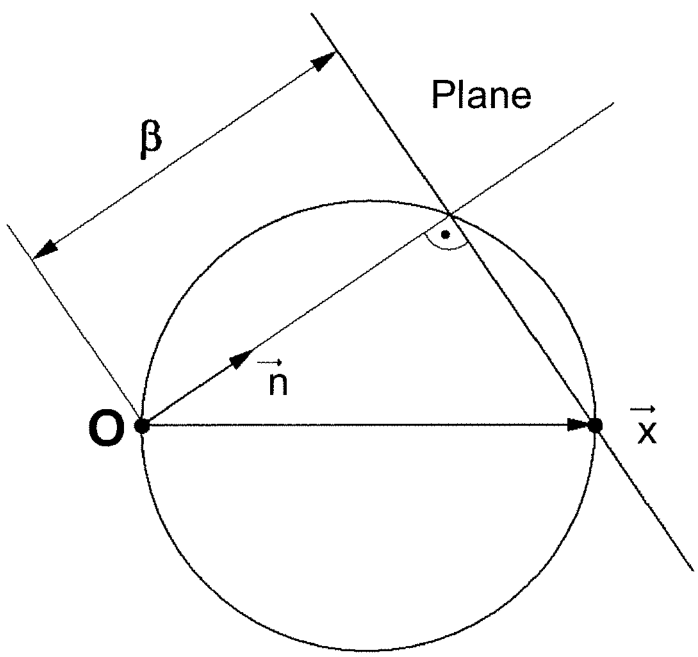

*Fig. 3*

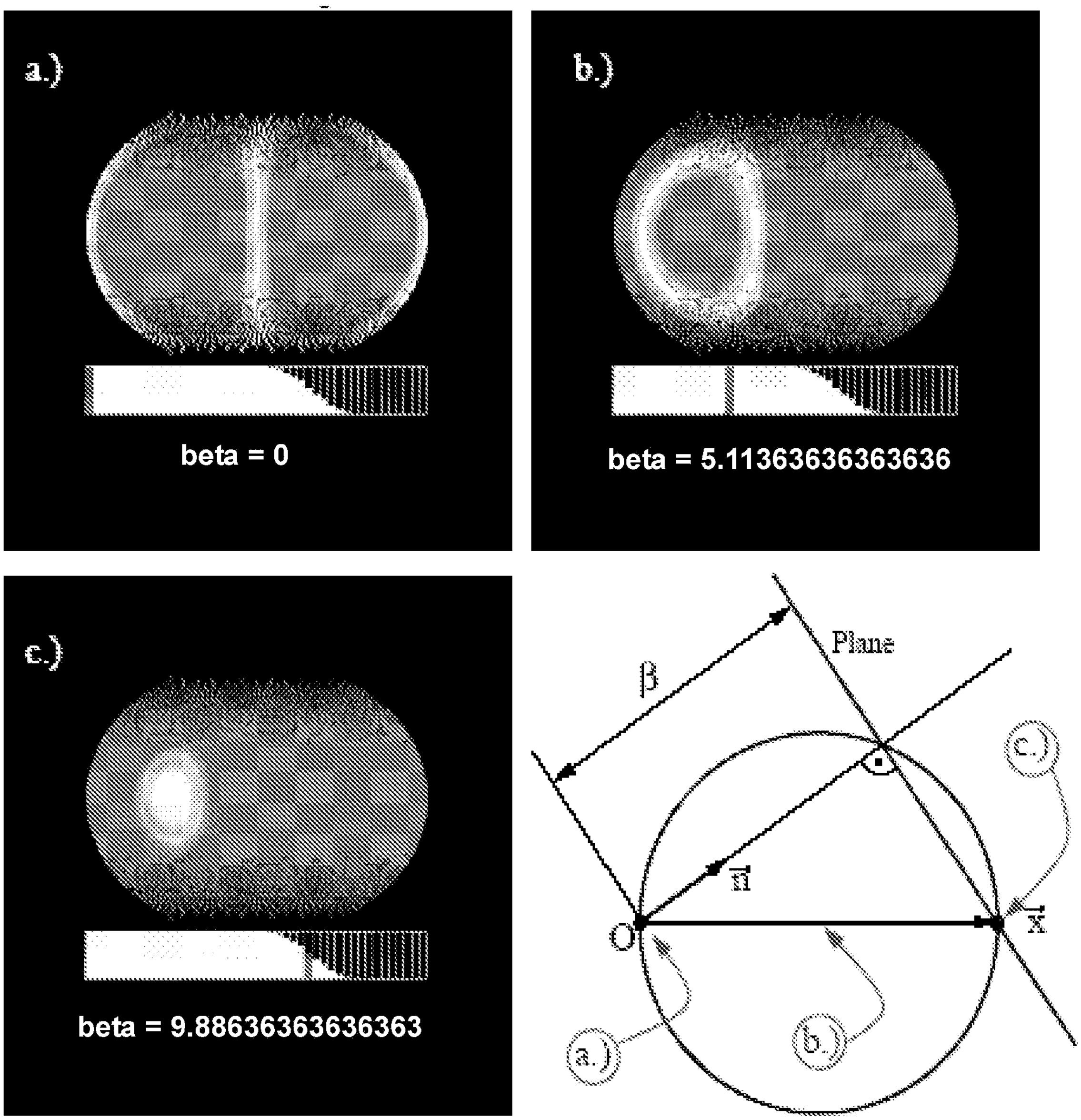
a.)
beta = 0
b.)
beta = 5.11363636363636
c.)
beta = 9.88636363636363
β
Plane
c.)
n
O
x
a.)
b.)

*Fig. 4*

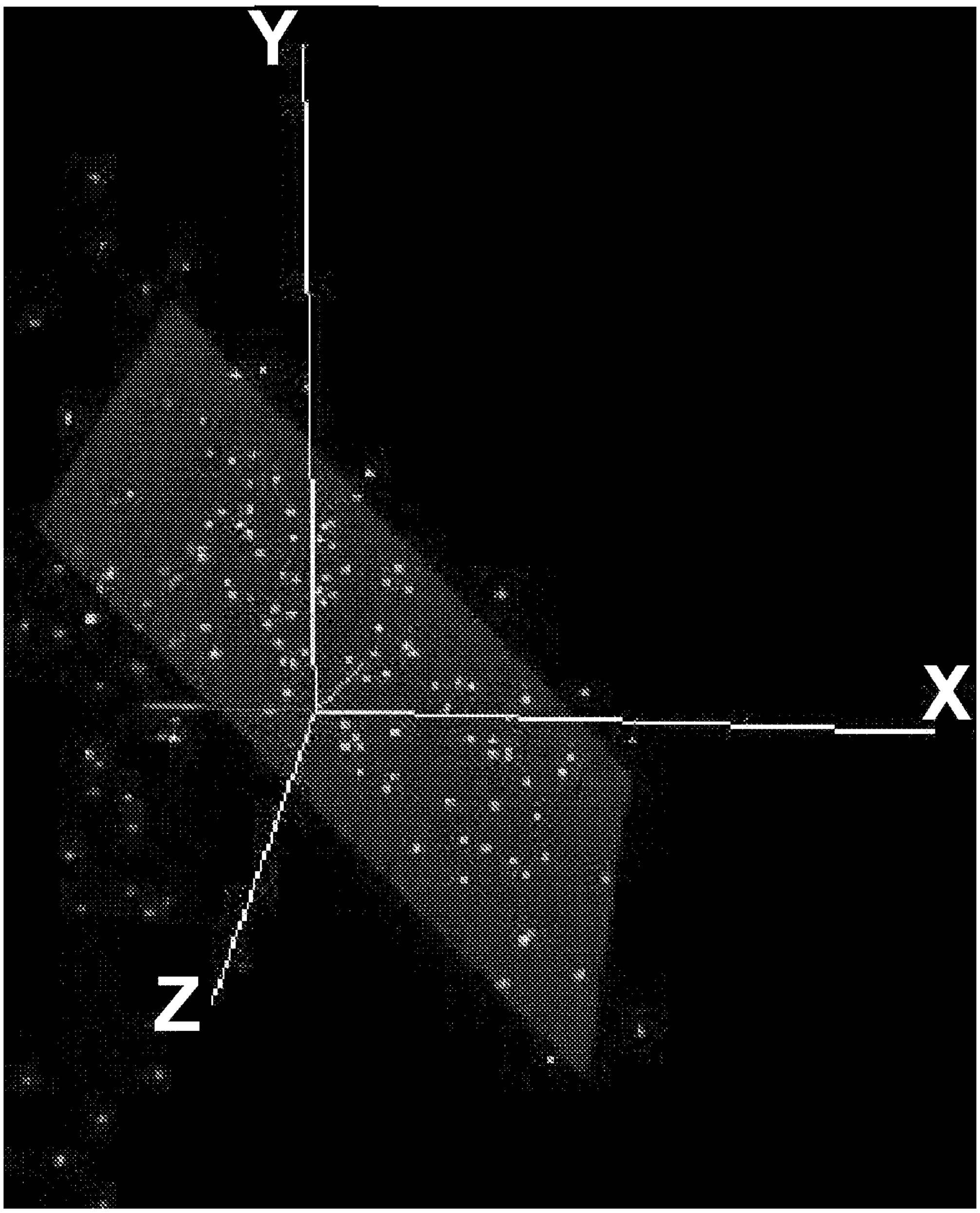

*Fig. 5*

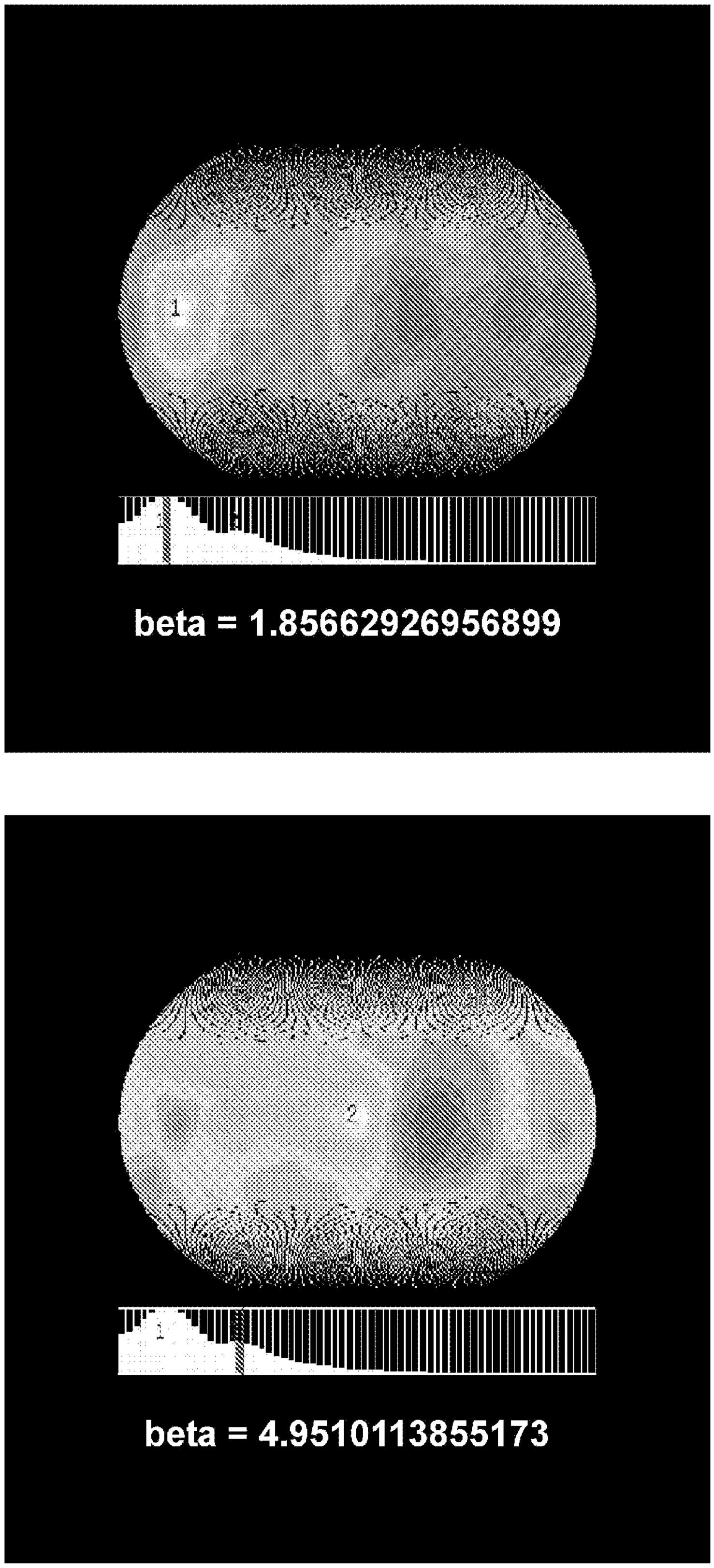

Fig. 6

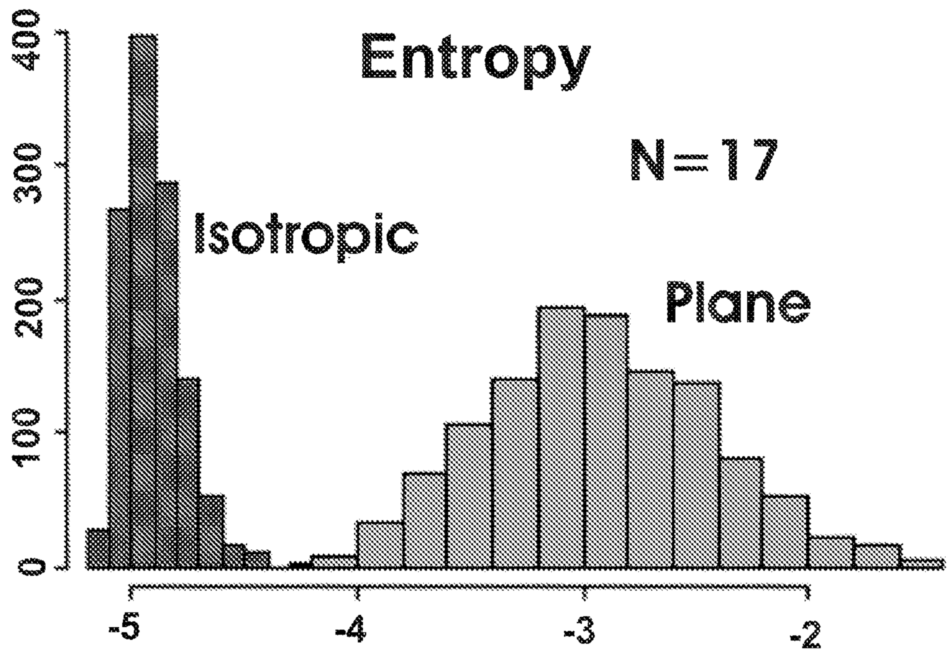
Entropy
N=17
Isotropic
Plane
400
300
200
100
0
-5
-4
-3
-2

Fig. 7

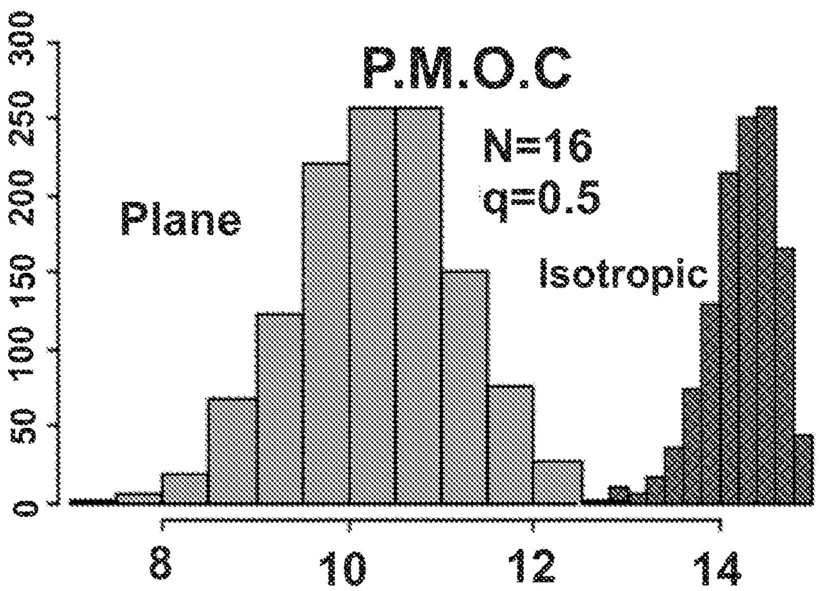
P.M.O.C
N=16
q=0.5
Plane
Isotropic
300
250
200
150
100
50
0
8
10
12
14

Fig. 8

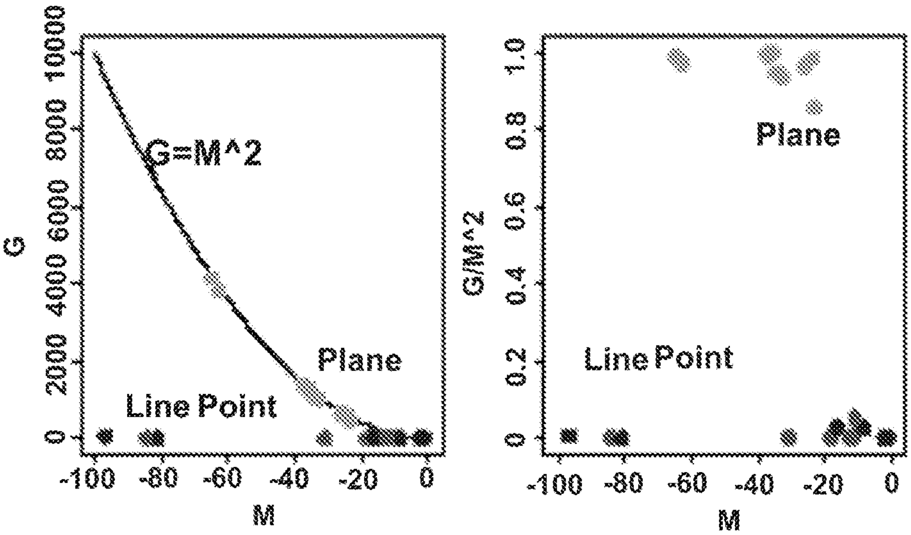
G
10000
8000
6000
4000
2000
0
G=M^2
Plane
Line Point
-100 -80 -60 -40 -20 0
M
G/M^2
1.0
0.8
0.6
0.4
0.2
0
Plane
Line Point
-100 -80 -60 -40 -20 0
M

Fig. 9

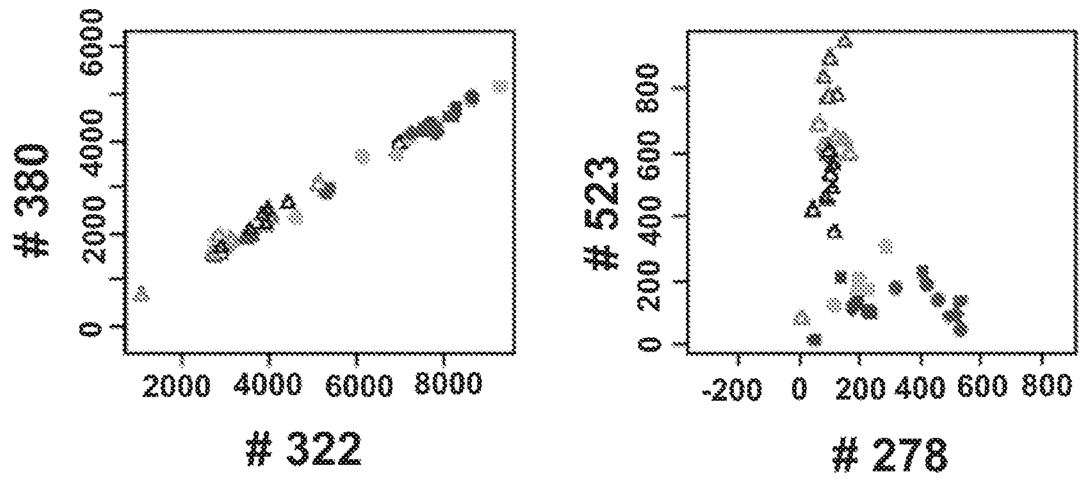
# 380
6000
4000
2000
0
2000 4000 6000 8000
# 322
# 523
800
600
400
200
0
-200 0 200 400 600 800
# 278

Fig. 10

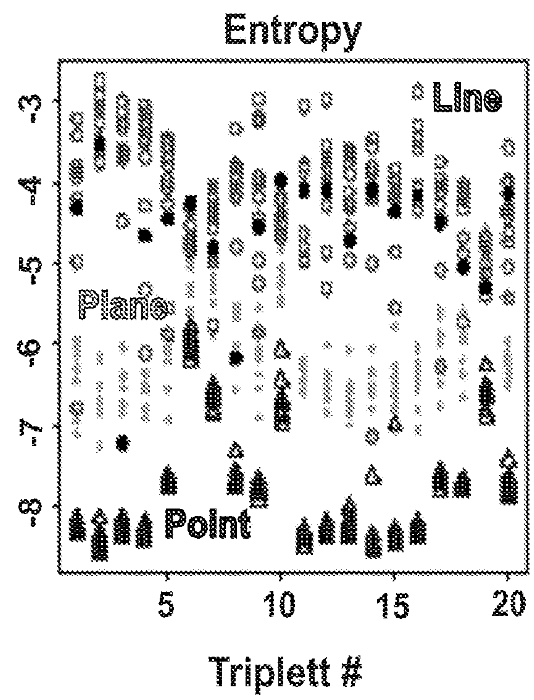
Entropy
Line
Plane
Point
-3
-4
-5
-6
-7
-8
5
10
15
20
Triplett #

Fig. 11

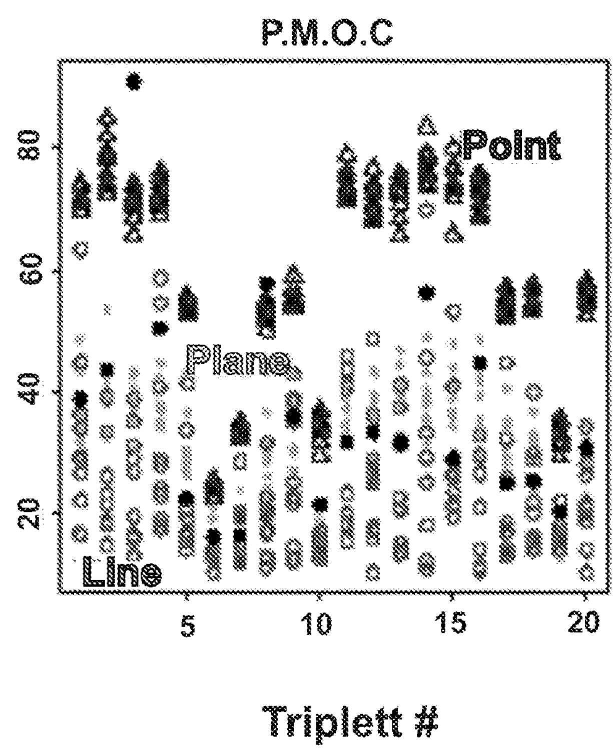
P.M.O.C
Point
Plane
Line
80
60
40
20
5
10
15
20
Triplett #

Fig. 12

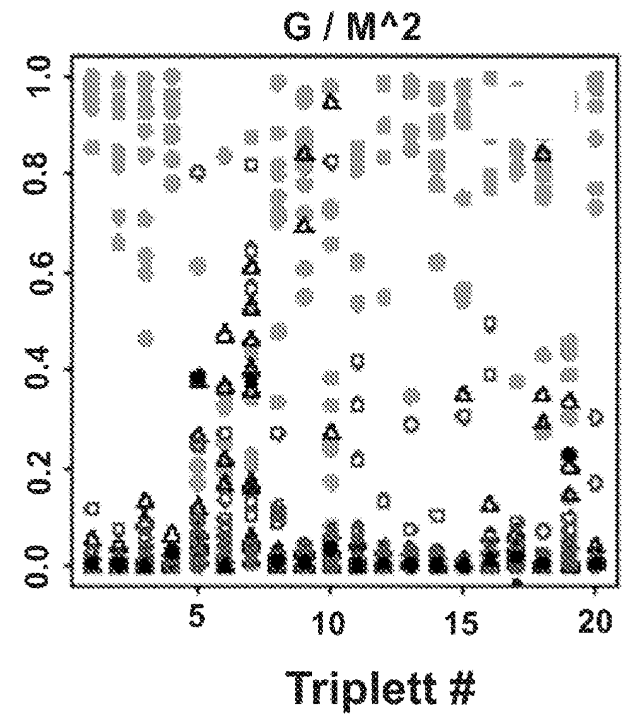
G / M^2
0.0
0.2
0.4
0.6
0.8
1.0
5
10
15
20
Triplett #

Fig. 13

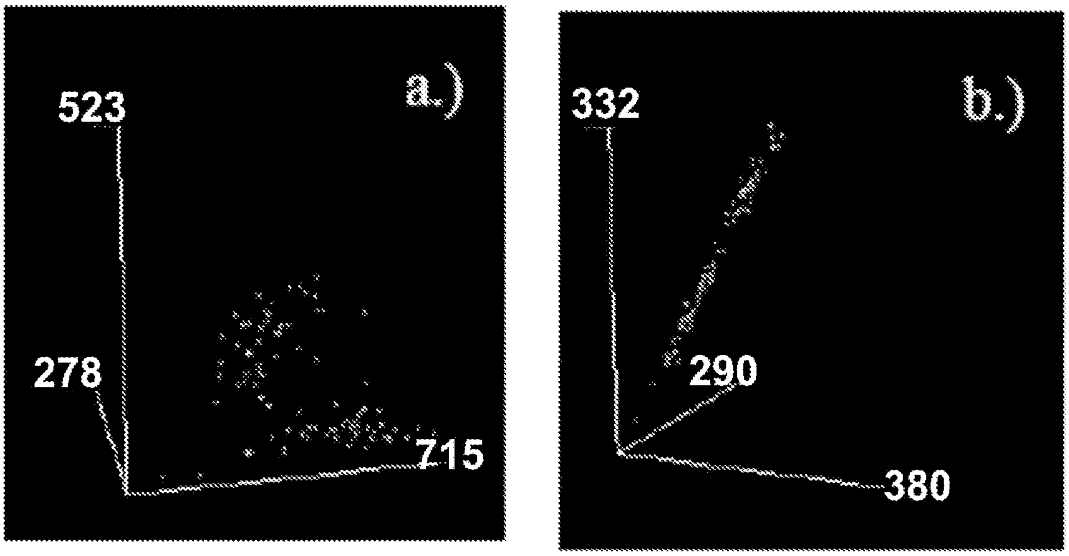
a.)
523
278
715
b.)
332
290
380

Fig. 14

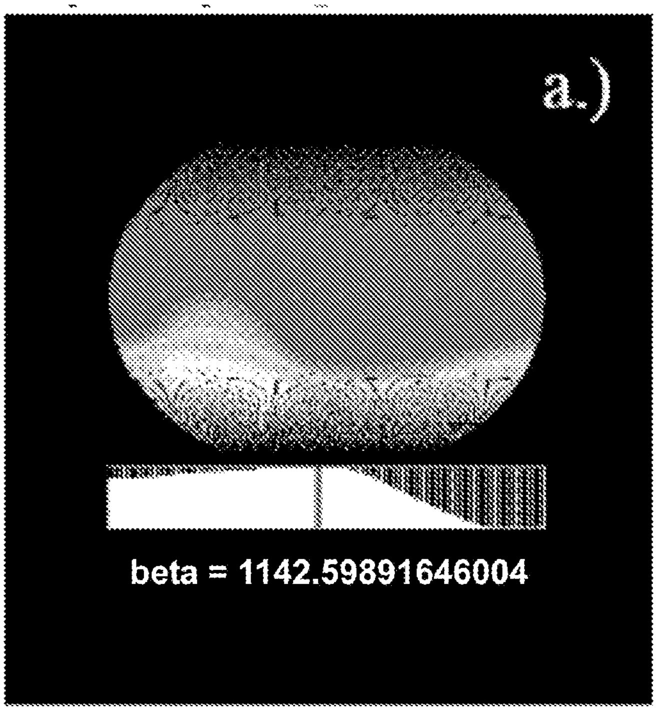


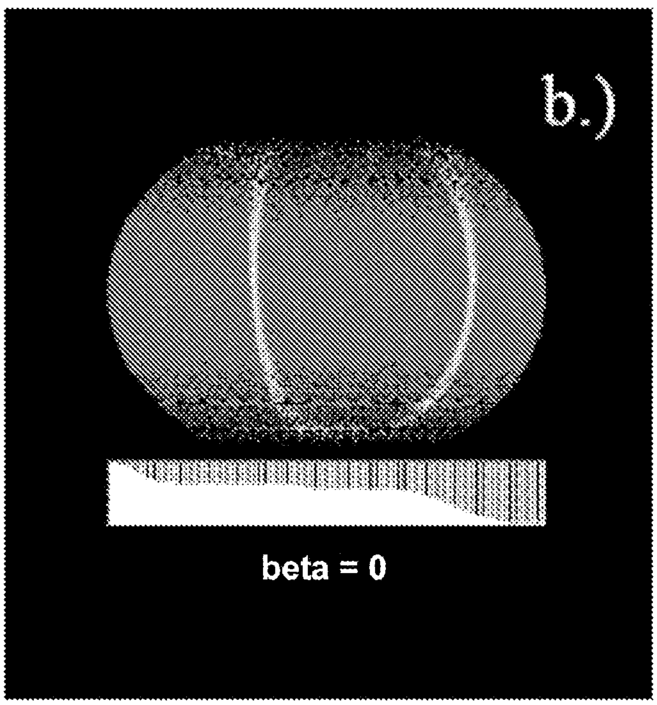

Fig. 15

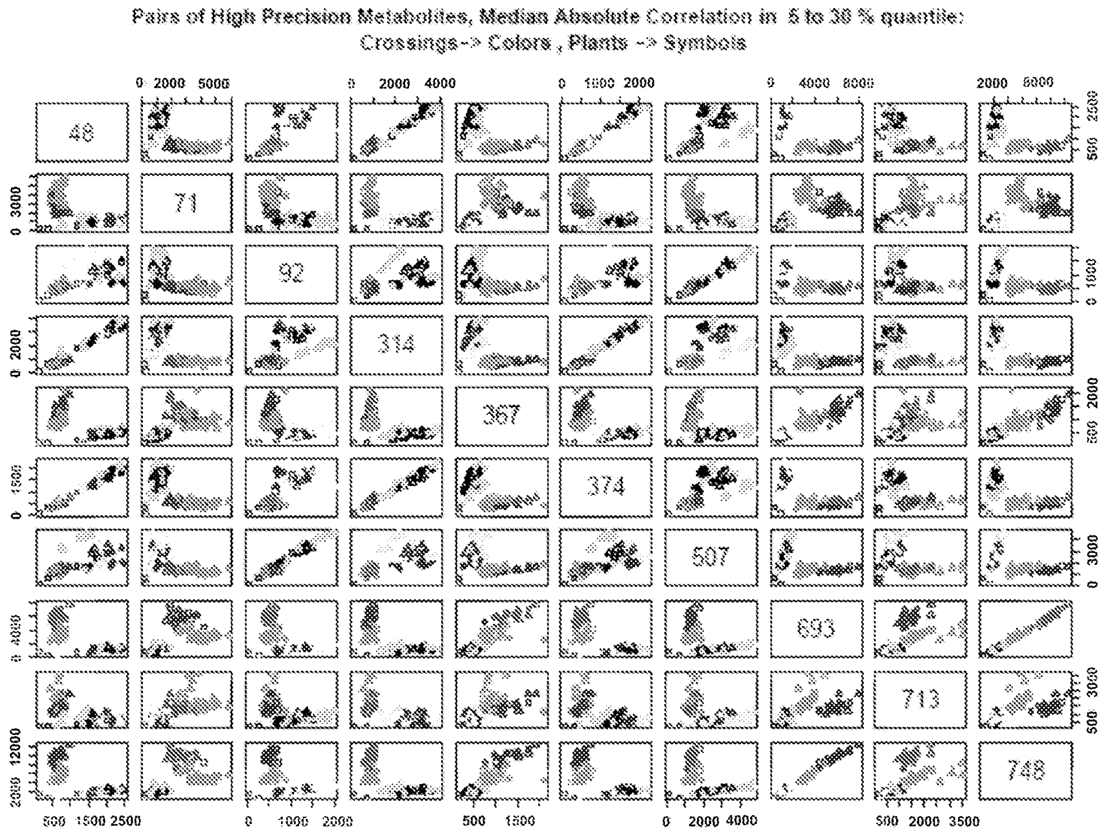
Pairs of High Precision Metabolites, Median Absolute Correlation in 5 to 30 % quantile:
Crossings-> Colors , Plants -> Symbols
48
71
92
314
367
374
507
693
713
748

Fig. 16

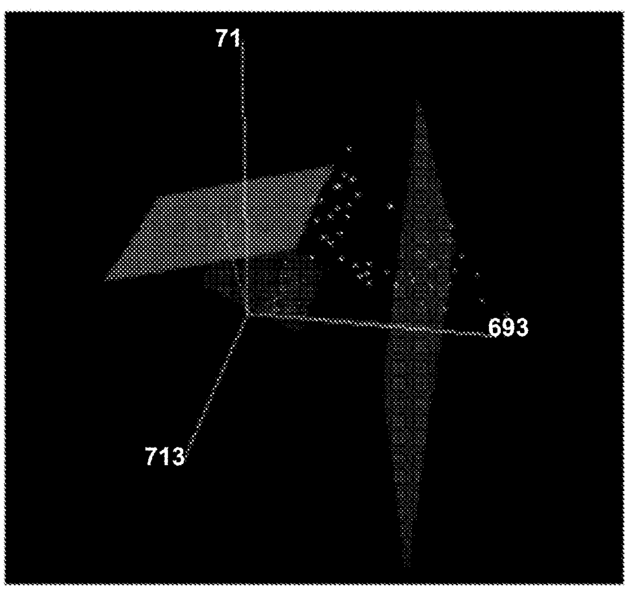
71
693
713

Europäisches Patentamt
European Patent Office
Office européen des brevets

# EUROPEAN SEARCH REPORT

Application Number
EP 10 16 0460

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X,D | KOSE FRANK ET AL: "Robust detection and verification of linear relationships to generate metabolic networks using estimates of technical errors." BMC BIOINFORMATICS 2007 LNKD- PUBMED:17517139, vol. 8, 2007, page 162, XP002607414 ISSN: 1471-2105 * the whole document * ----- | 1-8 | INV. G06F19/00 |
| X,D | WENTAO ZHAO ET AL: "Inferring Connectivity of Genetic Regulatory Networks Using Information-Theoretic Criteria" IEEE/ACM TRANSACTIONS ON COMPUTATIONAL BIOLOGY AND BIOINFORMATICS, IEEE SERVICE CENTER, NEW YORK, NY, US, vol. 5, no. 2, 1 April 2008 (2008-04-01), pages 262-274, XP011202168 ISSN: 1545-5963 * the whole document * ----- | 1-8 | |
| | | | TECHNICAL FIELDS SEARCHED (IPC) |
| | | | G06F |

The present search report has been drawn up for all claims

1

| Place of search | Date of completion of the search | Examiner |
|---|---|---|
| The Hague | 28 October 2010 | Swarén, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Grünenfelder B ; Winzeler E.** Treasures and Traps in Genome-Wide Datasets: Case Examples from Yeast. *Nat. Rev. Genetics,* 2002, vol. 3, 653-661
- **Fiehn O ; Kopka J ; Dörmann P ; Altmann T ; Trethewey R ; Willmitzer L.** Metabolite Profiling for Plant Functional Genomics. *Nat. Biotechnol.,* 2000, vol. 18, 1157-1161
- **Pandey A ; Mann M.** Proteomics to Study Genes and Genomes. *Nature,* 2000, vol. 405, 837-846
- **Heinrich R ; Schuster S.** The Regulation of Cellular Systems. Chapman and Hall, 1996
- **Jamshidi N ; Palsson B.** Formulating Genome-Scale Kinetic Models in the Post-Genome Era. *Molecular Systems Biology,* 2008, vol. 4, 171
- **Stelling J ; Klamt S ; Bettenbrock K ; Schuster S ; Gilles E.** Metabolic Network Structure Determines Key Aspects of Functionality and Regulation. *Nature,* 2002, vol. 420, 190-193
- **Butte A ; Kohane IS.** Mutual Information Relevance Networks: Functional Genomic Clustering Using Pair-Wise Entropy Measurements. *Pac. Symp. Biocomput.,* 2000, vol. 5, 415-426
- **Kose F ; Budczies J ; Holschneider M ; Fiehn O.** Robust detection and verification of linear relationships to generate metabolic networks using estimates of technical errors. *BMC Bioinformatics,* 2007, vol. 8, 162
- **Steuer R ; Kurths J ; Daub C ; Selbig J.** The Mutual Information: Detecting and Evaluating Dependencies Between Variables. *Bioinformatics,* 2002, vol. 18 (2), 231-240
- **Margolin A ; I Neemenmann KB ; Wiggins C ; Stolovitzky G ; Favera RD ; Califano A.** Arcane: An Algorithm for the Reconstruction of Gene Regulatory Networks in a Mammalina Cellular Context. *BMC Bioinformatics,* 2006, vol. 7 (1), 7
- **Yang Y ; Tashman A ; Lee J ; Yoon S ; Mao W ; Ahn K ; Kim W ; Mendell N ; Gordon D ; Finch S.** Mixture Modelling of Microarray Gene Expression Data. *BMC Proc,* 2007, vol. 1 (1), 50
- **Zhao W ; Serpedin E ; Gougherty E.** Inferring Connectivity of Genetic Regulatory Networks Using Information-Theoretic Criteria. *IEEE/ACM Transactions on Computational Biology and Bioinformatics,* 2008, vol. 5, 262-274
- **Scholz M ; Gatzek S ; Sterling A ; Fiehn O ; Selbig J.** Metabolite fingerprinting: detecting biological features by independent component analysis. *Bioinformatics,* 2004, vol. 20 (15), 2447-2454
- Bayesian estimation of faults geometry based on seismic catalogue data. *EOS Trans. Amer. Geophys. Union, Fall Meet.,* 2006, vol. 87
- **Bronstein I ; Semendjajew K.** Taschenbuch der Mathematik. Thun und Frankfurt am Main. Verlag Harri Deutsch, 2001
- **Jaynes E.** The Well-Posed Problem. *Foundations of Physics,* 1973, vol. 3 (4), 477-491
- Probability Theory. **Jaynes E ; Bretthorst G.** Logic of Science: Theory and Elementary Applications. Cambridge University Press, 2003, vol. 1
- **Freeden W ; Gervens T ; Schreiner M.** Constructive Approximation on the Sphere: With Applications to Geomathematics. Claredon Press, 1998
- **Rinne H.** Taschenbuch der Statistik 2. Verlag Harri Deutsch, 1997
- **Hastie T ; Tibshirani R ; Friedman J.** The Elements of Statistical Learning. Springer, 2001
- **Gilks W ; Richardson S ; Spiegelhalter D.** Markov Chain Monte Carlo in Practice. Chappman & Hall, 1996